# EUROPEAN PATENT APPLICATION

(11) **EP 1 995 306 A1**
(43) Date of publication of application: **26.11.2008**
(21) Application number: 07707624.8
(22) Date of filing: 29.01.2007
(51) Int. Cl.: C12N 1/20, C12P 7/64, A61K 8/55, C12N 15/00, C12R 1/01

(54) **NOVEL MICROBE, LIPID MODIFYING AGENT, PROCESS FOR PRODUCING 2-ACYL-LYSOPHOSPHOLIPID, PROCESS FOR PRODUCING DIACYLGLYCEROL, PROCESS FOR PRODUCING CERAMIDE, AND METHOD OF DEGUMMING OIL OR FAT**

(30) Priority: 27.02.2006 JP 2006049648
(71) Applicant: Tokyo University of Marine Science and Technology, Tokyo 108-8477 (JP)
(72) Inventor: YAMAGUCHI, Kohji, Yokohama-shi, Kanagawa 223-0062 (JP); YAZAWA, Kazunaga, Fujisawa-shi, Kanagawa 251-0038 (JP); NISHIHARA, Masaaki, Hachiouji-shi, Tokyo 192-0354 (JP); IWASAKI, Jun, Hanno-shi, Saitama 357-0041 (JP); KAMATA, Masazumi, Hachiouji-shi, Tokyo 192-0914 (JP)
(74) Representative: Fiener, Josef
(86) International application number: PCT/JP2007/051393
(87) International publication number: WO 2007/097160

(57) **Abstract**

A novel supply source of enzyme being useful for modification of phospholipids, etc.; a process for producing 2-acyl-lysophospholipid; a process for producing monoacylglycerol; a process for producing a ceramide; and a novel method of degumming an oil or fat. There is provided a novel microbe belonging to the genus Moritella sp. and capable of producing an enzyme having phospholipase A₁ activity, phospholipase C activity, lysophospholipase C activity and sphingomyelin-lytic activity. By the use of this enzyme, phospholipids are hydrolyzed to thereby produce 2-acyl-lysophospholipid and diacylglycerol, and sphingomyelins are hydrolyzed to thereby produce ceramides. Further, an oil or fat is degummed.

## Description

### Field of the Invention

The present invention relates to a novel microorganism belonging to Moritella species, which is capable of producing enzymes having: phospholipase A₁ activity; phospholipase C activity; lysophospholipase C activity; and sphingomyelinase activity. Further, the invention relates to: a lipid-modifying agent containing an effective dose of the enzymes produced by such novel microorganism; a method for degumming fat and oil by use of such lipid-modifying agent in order to permit use thereof for lipid biochemical researches; and a method for producing 2-acyl lysophospholipid.

Phospholipases are enzymes workable for hydrolyzing ester bonds in phospholipids. Increased findings on important roles of such phospholipases in the metabolism of the phospholipids have revealed a wide existence and distribution of the phospholipases among the eukaryotic and prokaryotic organisms. Biochemically, the phospholipases affect metabolism in the membrane of organism and also affect construction and reconstruction of the membrane, while being implicated in a signaling cascade as well.

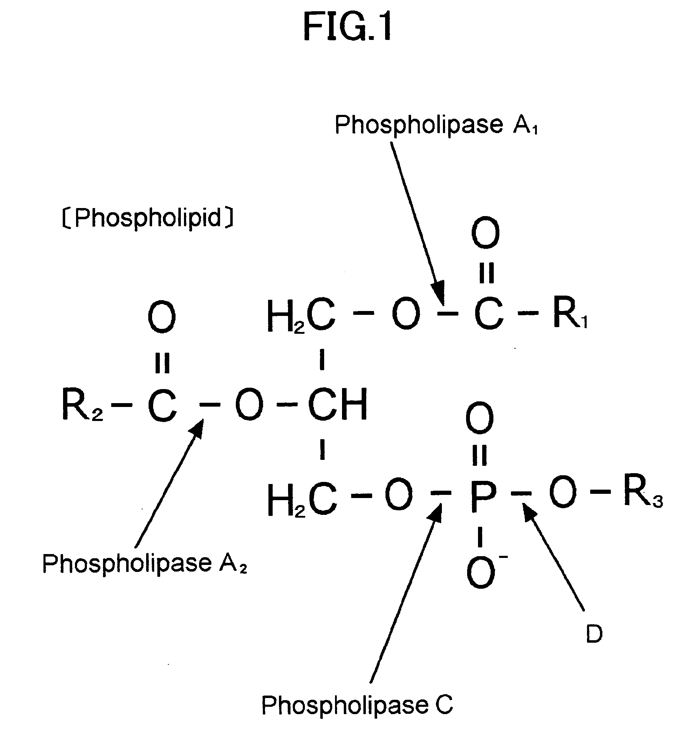

Some different types of the phospholipases are known according to their own specifities for targeting the corresponding ester bond points in phospholipid. For example, as understandable from Fig. 1, phospholipase A₁ hydrolyzes the ester bond of glycerophospholipid at the sn-1 position, thereby producing free fatty acid and 2-acyl lysophospholipid. Also, as in the Fig. 1, phospholipase A₂ hydrolyzes the ester bond of glycerophospholipid at the sn-2 position, thereby producing free fatty acid and 1-acyl lysophospholipid. Those phospholipaes A₁ and A₂ may be found either intracellularly or extracellularly, with a membrane-bound property and with a soluble property. Among them, an intracellular type of phospholipase A₂ is found in nearly all sorts of animals of higher orders. Phospholipase C is shown as hydrolyzing glycerophosphate, in which case, 1,2-diacylglycerol and phosphate radical are to be produced therefrom.

Recently, many kinds of degumming processes have been utilized to degum fat and oil. In the degumming processes, the above-stated enzymes, namely phospholipases, are employed. For example, the phospholipases A₁ and A₂ have been used commercially for degumming fat and oil, in various manners, which is known for example from the literature, "ENZYMAX_{™} Degumming", (published by Lurgi Life Science Technologies GmbH, Germany). The phospholipase C has also received attention and has been studied on its usage for the degumming, because the phospholipase C reacts with phospholipid to produce phosphate residue which is highly soluble in water, thus allowing for easy removal of that particular phosphate residue, and further, diglyceride, together with fat and oil, acts to reduce a loss caused by accumulation of the phosphate residue. (For example, see Dahlke (1998) "An enzymatic process for the physical refining of seed oils," Chem. Eng. Technol. 21:278-281; Clausen (2001) "Enzymatic oil degumming by a novel microbial phospholipase," Eur. J. Lipid Sci. Technol. 103:333-340.)

It is known that lysophospholipid is greater in surface-active property than normal phospholipids and therefore is a notable mode of lipid for allowing its effective use in foods. Namely, the lysophospholipid may be used for improving the emulsification stability, texture and elasticity of food to a satisfied degree, as compared with normal phospholipids. This may in turn reduce the amount of other required emulsifiers in making the food. Further, besides the foods, the lysophospholipid is valued in terms of its usability for cosmetic emulsifiers.

A typically known process for preparation of the lysophospholipid utilizes phospholipase A₂. At present, a phospholipase A₂ derived from porcine pancreas can only be used for industrial purposes. But, it has been found that, as compared with an emulsion by 1-acyl lysophospholipid produced by that phospholipase, an emulsion by 2-acyl lysophospholipid is much advantageous for practical use, because the emulsion by 2-acyl lysophospholipid is significantly low in isolation rate of oil layer relative to the emulsion by 1-acyl lysophospholipid. (see Lecture number P10, the 35th Oil Chemistry Symposium)

Phospholipases do not hydrolyze triglyceride, and therefore, even when the phospholipases act on a substrate containing triglyceride therein, neither of diglyceride and monoglyceride is generated therefrom. Thus, production and refining of lysophospholipid are possible without being inhabited by such glycerides, because the lysophospholipid itself is produced from hydrolysis of phospholipid by phospholipase. There has been developed a process for preparing phospholipase A₁ derived from fungus, such as filamentous fungus, but, it requires cultivation of strain for a long period of time and also requires troublesome operations for extracting the corresponding enzyme from a culture medium which contains bacterial bodies therein.

Phospholipase A₁ exists in animal's pancreases and livers as well as in microorganisms, and cooperates with phospholipase A₂ to assist in metabolic turnover of phospholipids. But, at present, the phospholipase A₁ is not available as a marketed product, inclusive of its refined product, in spite of its being a useful enzyme for analysis of phospholipid distributions in fatty acid molecule and for lipid biochemical researches.

Lysophospholipase C acts to remove phosphophate residues from 1- and 2- acyl lysophospholipids so as to produce monoacylglycerol and phosphophate base. Hence, 1-, and 2- acyl lysophospholipids, each having a known composition of fatty acid therein, may be reacted with the lysophospholipase C in order to obtain monoacylglycerol having a known fatty acid composition. Accordingly, the lysophospholipase C is also expected as an enzyme useful for lipid biochemical researches.

Sphingomyelin may be transformed specifically by the phospholipase C or sphingomyelinase into a ceramide. The ceramide so produced can be utilized as a skin moisturizer element, for instance.
Relevant patent literature 1: Japanese Laid-Open Patent Publication No. Hei 10-323182
Relevant patent literature 2: Japanese Laid-Open Patent Publication No. Hei 11-228986
Relevant patent literature 3: Japanese Laid-Open Patent Publication No. Hei 11-318434

### Disclosure of the Invention

### Technical Solutions Aimed By The Invention

It is a purpose of the present invention to provide the following technical solutions: (1) a new source for providing enzymes useful for degumming fat and oil and modifying phospholipids as well as for lipid biochemical researches, and so forth, wherein such enzymes comprise: phospholipase A₁ ; phospholipase C; lysophospholipase C; and sphingomyelin degradation enzyme (sphingomyelinase) ; (2) methods for permitting use of those enzymes for degumming purpose in the process of producing fat and oil, and also permitting use of the enzymes for preparation of lysophospholipid; (3) a method for preparing monoacylglycerol by use of the lysophospholipase C; and (4) a method for preparing ceramide by use of the sphingomyelinase.

### Means for Achieving the Technical Solutions

In order to achieve the above-stated purpose and technical solutions, the present invention provides a novel microorganism (HFHI-0014) which belongs to Moritella species and is capable of producing enzymes having the respective following activities: a phospholipase A₁ activity; a phospholipase C activity; a lysophospholipase C activity; and a sphingomyelinase activity, and also the present invention provides a lipid-modifying agent containing effective dose of the enzymes secreted from the novel microorganism.

Namely, an enzyme with phospholipase A₁ activity, produced by the novel microorganism, may be used to hydrolyze phospholipid to obtain 2-acyl lysophospholipid. An enzyme with phospholipase C activity, produced by the novel microorganism, may be used to hydrolyze phospholipid to obtain diacylglycerol. An enzyme with sphingomyelinase activity, produced by the novel microorganism, may be used to hydrolyze sphingomyelin to obtain ceramide. Further, the enzymes produced by the novel microorganism may be used to hydrolyze phospholipids present in fat and oil, thus permitting use of the enzymes for degumming the fat and oil.

The aforementioned novel microorganism also has bacterial properties as well as physiological and biochemical properties, all of which will be set forth later. It is noted that a base sequence of 16Sr DNA gene in the novel microorganism is described in the sequence listing for the HFHI-0014 strain under the sequence number 1, the sequence listing having been filed together with the present specification. It is also noted that this novel microorganism in the present invention has been domestically deposited in the National Institute of Advanced Industrial Science and Technology, International Patent Organism Depositary, under a strain identification name of HFHI-0014 and granted a deposit number of FERM AP-20806. Thereafter, such domestically deposited novel microorganism has been transferred on January 22, 2007 to international depositary and granted a deposit number of FERM BP-10766. The previously stated enzymes produced by that novel microorganism may be practically used in a manner contained in bacterial body of the microorganism and/or in culture solution.

The culture solution stated above contains all culture products obtained through cultivation of the strain Moritella sp. HFHI-0014, irrespective of whether the bacterial bodies of the microorganism may or may not be present in the culture solution. According to the present invention, preparation of such culture solution may for example be based on usual steps of cultivating the strain in a culture medium containing peptone and yeast extract therein, and thereafterr subjecting the culture medium to purification through commonly practiced method for removal of the bacterial bodies therefrom. In the present invention, considering subsequent preparation steps and enzyme production processes, it is preferred to remove the bacterial bodies from the culture medium by centrifuge after the cultivation, so that a culture solution (or culture supernatant) containing the foregoing enzymes is obtained, without any bacterial body therein. Proper substrates are provided for the respective enzymes, and for example, a substrate suited for the phospholipase A₁ and phospholipase C may be a phospholipid having an ester bond at each of the sn-1 and sn-2 positions therein, wherein such phospholipid is either one of chemically or enzymatically prepared phospholipids, or one of naturally existing phospholipids derived for example from: plants such as soybeans and rapeseeds; land and marine animals such as salmon roe, squid and egg yolk; and microorganisms such as bacteria and yeast.

### Advantageous Effects of the Invention

In accordance with the present invention, it is effectively possible to provide a culture solution having enzymatic activities useful for lipid modification (i.e. lipid-modifying agent) and for lipid biochemical researches, wherein the enzymatic activities are: phospholipase A₁ activity; phospholipase C activity; lysophospholipase C activity; and sphingomyelinase activity.

Further, in the present invention, even at the temperature of as low as 10 degrees Celsius, the foregoing enzymatic activities are still effective to act on phospholipids to produce 2-acyl lysophospholipid, diacylglycerol, and monoacylglycerol, and also effective to act on sphingomyelin to produce ceramide. This advantageously prevents alteration and deterioration of lipids and proteins at the process where the lipids and proteins undergo oxygen treatment, since such alteration and deterioration may easily occur subject to changes of temperature.

### Brief Description of the Drawings

Fig. 1 is a diagram for showing points of phospholipid which are to be hydrolyzed by phospholipases.
Fig. 2 is a chart showing the results of molecular phyloanalysis of the strain of novel microorganism of the present invention, which are prepared on the basis of deduction from homology searches conducted against reference strain database of bacteria as well as from top 30 base sequences of high homology rate obtained from the homology searches.

### Best Mode for Carrying Out the Present Invention

First of all, the novel microorganism (HFHI-0014) in the present invention had been cultivated in a K28 culture medium to be described later, for five days, under aerobic conditions and at the temperature of 10 degrees Celsius. Observation and bacterial property test had been conducted thereon during the cultivation, and the results were shown in Table 1 below.

**Table 1**

| Results of Bacterial Property Test for *Moritella sp.* HFHI-0014 strain | | |
|---|---|---|
| Test items | Results | |
| | | |
| Cultivation temperature (degrees Celsius) : | | 10 degrees |
| Cell Shape: | polymerismic rod bacteria (1.0 to 1.2 x 2.0 to 3.0 | |
| micrometers) | | |
| Gram Stainability: | | - |
| Presence of spores: | | - |
| Mobility: | | + |
| Colony state and shape: | Culture time spent: | 48 hours |
| | Diameter: | 1.0 to 2.0 mm |
| | Color tone: | cream |
| | Shape: | circular |
| | Topography: | lens-shaped |
| | Fringe: | whole border |
| | Surface shape: | smooth |
| | Transparency: | semi-transparent |
| | Viscosity: | butter-like |

| Growth temperature test: | | |
|---|---|---|
| (degrees Celsius) | at 20 degrees | - |
| | at 15 degrees | +w |
| Catalase reaction: | | + |
| Oxidase reaction: | | + |
| Fermentation of glucose: | | + |
| O/F test (Oxidation/Fermentation): | | +/+ |
| Growth under aerobic conditions: | | + |

| | | |
|---|---|---|
| (Note: the term "+w" means "weakly positive") | | |

Accordingly, it is to be seen that the microorganism (HFHI-0014) of the present invention is a polymerismic rod bacterial with mobility, which is negative in gram stainability and capable of fermenting glucose, and that both catalase and oxidase reactions thereof are found positive.

Also, the microorganism (HFHI-0014) was tested in terms of its physiological and biochemical properties, and the results are shown in Table 2 below.

**Table 2**

| Results of Physiological and Biochemical Property Test for *Moritella sp.* HFHI-0014 strain | | | |
|---|---|---|---|
| Substrate, reaction and enzyme activity | Results | Substrate, reaction and enzyme activity | Results |
| | | | |
| Nitrate reduction | + | D-Mannitol | - |
| Indole production | - | N-acetyl-D-glucosamine | - |
| Glucose acidification | - | Maltose | - |
| Arginine dihydrolase | - | Potassium gluconate | - |
| Urease | - | n-Capric acid | - |
| Esculin hydrolysis | - | Adipic acid | - |
| Gelatin hydrolysis | - | d1-Malic acid | - |
| Beta galactosidase | - | Sodium citrate | - |
| Glucose | - | Phenyl acetate | - |
| L-Arabinose | - | Cytochrome oxidase | + |
| D-Mannose | + | Xylose | - |
| Cellobiose | + | Glycerol | - |
| Starch hydrolysis | - | D-Galactose | - |

Determinations were done by AIP kits for the foregoing test, as a result of which, it is seen that the enzymes produced by the microorganism (HFHI-0014) of the present invention reduced nitrate and oxidized both of cellobiose and D-mannose, but did not demonstrate any arginine dihydrolase activity.

Analysis was effected for the base sequence of 16Sr DNA gene in the microorganism (HFHI-0014), with the result that a whole of the 16Sr DNA base sequence was obtained, as shown in the sequence listing under the sequence number 1.

Further, homology searches were conducted for the results of the aforementioned analysis of 16Sr DNA base sequence. Results of the homology searches were obtained as shown in Tables 3 and 4 below. Note that the Table 3 shows results of homology search conducted against a reference strain database for bacteria, whereas the Table 4 shows results of homology search conducted against an international base sequence database.

**Table 3**

| Results of Homology Search for *Moritella sp.* HFHI-0014 against Reference Strain | | |
|---|---|---|
| Database | Bacterial species name | Strain name |
| Homology rate (%) | | |
| *Moritella marina* | ATCC15381 | 98.3 |
| *Moritella purofunda* | 2674 | 98.3 |
| *Moritella viscosa* | NVI 88/478 | 98.2 |
| *Moritella japonika* | DSKI | 98.1 |
| *Moritella abyssi* | 2693 | 98.3 |
| *Moritella yayanosii* | DB21 MT-5 | 97.8 |
| *Shewanella waksmanii* | KMM 3823 | 92.6 |
| *Shewanella gelidimarina* | ACAM456 | 91.6 |
| *Shewanella fidelis* | KMM 3582 | 91.9 |
| *Shewanella baltica* | NTCT 10735 | 91.5 |
| *Shewanella violacea* | DSS12 | 91.5 |
| *Shewanella affinis* | KMM 3587 | 91.2 |
| *Shewanella marisflavi* | SW-117 | 91.1 |
| *Vibrio penaecida* | DSM 14398 | 91 |
| *Shewanella oneidensis* | MR-1 | 90.6 |
| *Photobacterium lipolyticum* | M37 | 91.1 |
| *Shewanella schlegeliana* | HRKA1 | 91.5 |
| *Shewanella alagae* | ATCC51192 | 90.6 |
| *Shewanella sairae* | SM2-1 | 91.3 |
| *Shewanella marinintestina* | IK-1 | 91.3 |
| *Shewanella denitrificans* | OS-217 | 89.9 |
| *Photobacterium frigidiphilum* | SL13 | 90.5 |
| *Shewanella woodyi* | MS32 | 91.2 |
| *Vibrio wodanis* | NVI 88/441 | 90.4 |
| *Shewanella aquimarina* | SW-120 | 90.5 |
| *Shewanella pealeana* | ANG-SQ1 | 90.2 |
| *Psychromonas antarctica* | star-1 | 90.4 |
| *Shewanella benthica* | ATCC 43992 | 91.2 |
| *Shewanella frigidimarina* | ACAM591 | 90 |
| *Shewanella gaetbuli* | TF-27 | 90.4 |

**Table 4**

| Results of Homology Search for *Moritella sp.* HFHI-0014 against International Base Sequence Database | | |
|---|---|---|
| Bacterial species name | Strain Name | Homology rate (%) |
| *Moritella sp.* | | 99.5 |
| *Moritella sp.* | 56AI | 99.4 |
| *Moritella sp.* | T4708 | 99.4 |
| *Moritella sp.* | T4702 | 99.4 |
| *Moritella sp.* | 36AI | 99.4 |

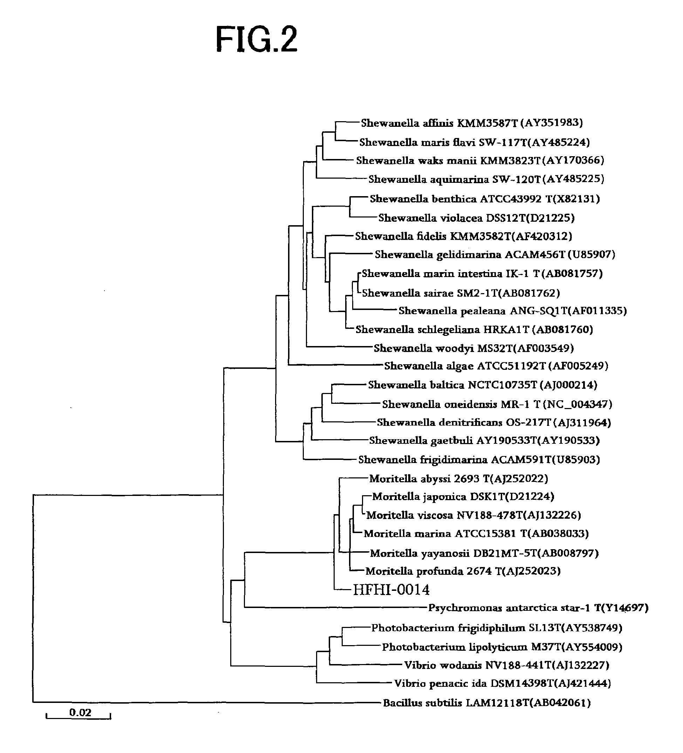

Molecular phyloanalysis was also effected on the basis of 30 base sequences respectively of top 30 strains which were extremely high in homology rate for the HFHI-0014 strain, wherein those 30 base sequences were obtained from the previously stated reference strain database. As a result thereof, deduced molecular phylogeny of the HFHI-0014 strain is shown in Fig. 2.

In the above-described manner, the data on 16Sr DNA base sequence of HFHI-0014 strain were checked by the homology searches conducted against the reference strain database of bacteria as well as against the international base sequence database. The results of those searches showed that the HFHI-0014 strain was highly homologous to Moritella species such as Moritella marina and Moritella purofunda. However, according to the search results, none of all known Moritella species was identical to the HFHI-0014 strain in terms of properties. Therefore, it can be determined that the HFHI-0014 strain is a novel Moritella species.

On February 22, 2006, the foregoing microorganism (HFHI-0014) of the present invention has been deposited in the National Institute of Advanced Industrial Science and Technology, International Patent Organism Depositary (at Tsukuba Central 6, 1-1, Higashi 1-chome, Tsukuba-shi, Ibaragi, Japan) and granted the deposit number of FERM AP-20806, after which, such domestically deposited novel microorganism has been transferred on January 22, 2007 to international depositary and granted the deposit number of FERM BP-10766.

### Exemplary Embodiments of the Present Invention

Hereinafter, a more specific description will be made of the present invention by way of plural examples of experiments. It is however noted that the invention is not limited to any of the experiments.

Experiment 1: The Moritella sp. HFHI-0014 strain was inoculated in the K28 culture medium (containing 0.5 % of peptone, 0.1 % of yeast extract, and 50% of artificial seawater), and subjected to shaking cultivation for 72 hours at the temperature of 10 degrees Celsius. Centrifugation was carried out to the thus-cultivated medium for removal of the bacterial bodies therefrom, whereupon a culture supernatant without the bacterial bodies was obtained. As a substrate, one of the following two substrates (two different synthesized PCs) was employed: 5 mg of 1-palmitoyl-2-oleoylphosphatidylcholine (16:0/18:1 (n-9)-PC); and 5 mg of 1-oleoyl-2-palmitoylphosphatidylcholine (18:1(n-9)/16:0-PC). On the other hand, 0.5 ml of diethyl ether was added to 0.5 ml of the afore-said culture supernatant, thereby providing a total 1.0 ml of culture solution. Then, those substrate and culture solution were agitated together for 13 hours at the temperature of 10 degrees Celsius, followed by extraction of lipids therefrom by Folch method. Silica gel TLC (thin layer chromatography) was used to analyze the lipids, with the result that there were obtained a fraction of lysophosphatidylcholine (LPC) and a fraction of monoacylglycerol (MG). The constituent fatty acids respectively of those LPC and MG were each obtained as a methyl ester through hydrogen chloride-methanol method. Thereafter, gas chromatography was performed for quantitative determination of each of such two methyl esters respectively associated with the LPC and MG. As a result thereof, as shown in Table 5 below, a data of fatty acid composition was obtained for each of the LPC and MG as well as for each of the two substrates stated above.

**Table 5**

| Fatty Acid Composition (mol%) of each of LPC and MG produced from Hydrolysis of Synthesized PC by use of Culture Supernatant of *Moritella sp.* HFHI-0014 Strain | | | | | |
|---|---|---|---|---|---|
| | | | | | (%) |
| | | 16:0/18:1 PC | | 18:1/16:0PC | |
| Strain | | | | | |
| | | C16:0 | C18:1 | C18:1 | C16:0 |
| | | | | | |
| | Synthesized PC: | 44.92 | 52.04 | 48.66 | 51.34 |
| HFHI0014 | | | | | |
| | LPC: | 99.34 | 5.1 | 89.78 | 0.66 |
| | MG: | 100 | 0 | 100 | 0 |

The results of the foregoing analysis indicated that the LPC had fatty acids at each of the sn-1 and sn-2 positions with respect to each of the foregoing two substrates used (synthesized PCs), but revealed many fatty acids remained at the sn-2 position in that LPC, which means that acyl group at the sn-1 position in the LPC was selectively hydrolyzed. Hence, it was confirmed that the aforementioned culture supernatant, used as an enzyme solution, actually demonstrated phospholipase A₁ activity, as a major enzymatic hydrolysis activity thereof. Further, the results of the analysis indicated that the MG had fatty acids at the sn-2 position only, with respect to both of the afore-said two substrates. This revealed the fact that 2-acyl lysophospholipid had been produced due to the foregoing phospholipase A₁ activity and thereafter hydrolyzed, which means that lysophospholipase C activity was actually done in the culture supernatant to act on the 2-acyl lysophospholipid.

Experiment 2: The Moritella sp. HFHI-0014 strain was inoculated in the K28 culture medium (containing 0.5 % of peptone, 0.1 % of yeast extract, and 50% of artificial seawater), and subjected to shaking cultivation for 72 hours at the temperature of 10 degrees Celsius. Centrifugation was carried out to the thus-cultivated medium for removal of the bacterial bodies therefrom, whereupon a culture supernatant without the bacterial bodies was obtained. As a substrate, 0.5 mg of soybean oil was provided. On the other hand, 0.5 ml of diethyl ether was added to 0.5 ml of the afore-said culture supernatant, thereby providing a total 1.0 ml of culture solution. Then, those substrate and culture solution were agitated together for 18 hours at the temperature of 10 degrees Celsius. A resultant solution obtained by such agitation was fractionated and analyzed by silica gel TLC, with the result that any free fatty acid was not detected therefrom, which confirmed that no hydrolysis was done in the culture supernatant stated above. Consequently, it was determined that the culture supernatant has no lipase activity to act on triglyceride.

Experiment 3: The Moritella sp. HFHI-0014 strain was inoculated in the K28 culture medium (containing 0.5 % of peptone, 0.1 % of yeast extract, and 50% of artificial seawater), and subjected to shaking cultivation for 72 hours at the temperature of 10 degrees Celsius. Centrifugation was carried out to the thus-cultivated medium for removal of the bacterial bodies therefrom, whereupon a culture supernatant without the bacterial bodies was obtained. As a substrate, 1.0 mg of diacylglycerol (DG) was provided. On the other hand, 0.5 ml of diethyl ether was added to 0.5 ml of the afore-said culture supernatant, thereby providing a total 1.0 ml of culture solution. Then, those substrate and culture solution were agitated together for 18 hours at the temperature of 10 degrees Celsius. A resultant solution obtained by such agitation was fractionated and analyzed by silica gel TLC, with the result that any of free fatty acid and MG was not detected therefrom, which confirmed that no hydrolysis was done in the culture supernatant. Consequently, it was determined that the culture supernatant has no lipase activity to act on the DG.

Experiment 4: The Moritella sp. HFHI-0014 strain was inoculated in the K28 culture medium (containing 0.5 % of peptone, 0.1 % of yeast extract, and 50% of artificial seawater), and subjected to shaking cultivation for 72 hours at the temperature of 10 degrees Celsius. Centrifugation was carried out to the thus-cultivated medium for removal of the bacterial bodies therefrom, whereupon a culture supernatant without the bacterial bodies was obtained. As a substrate, 1.0 mg of MG was provided. On the other hand, 0.5 ml of diethyl ether was added to 0.5 ml of the afore-said culture supernatant, thereby providing a total 1.0 ml of culture solution. Then, those substrate and culture solution were agitated together for 18 hours at the temperature of 10 degrees Celsius. A resultant solution obtained by such agitation was fractionated and analyzed by silica gel TLC, with the result that any free fatty acid was not detected therefrom, which confirmed that no hydrolysis was done in the culture supernatant. Consequently, it was determined that the culture supernatant had no lipase activity to act on the MG.

Experiment 5: The Moritella sp. HFHI-0014 strain was inoculated in the K28 culture medium (containing 0.5 % of peptone, 0.1 % of yeast extract, and 50% of artificial seawater), and subjected to shaking cultivation for 72 hours at the temperature of 10 degrees Celsius. Centrifugation was carried out to the thus-cultivated medium for removal of the bacterial bodies therefrom, whereupon a culture supernatant without the bacterial bodies was obtained. As a substrate, 1.0 mg of egg yolk (PC) was provided. On the other hand, 0.5 ml of diethyl ether was added to 0.5 ml of the afore-said culture supernatant, thereby providing a total 1.0 ml of culture solution. Then, those substrate and culture solution were agitated together for 13 hours at the temperature of 10 degrees Celsius. A resultant solution obtained by such agitation was fractionated and analyzed by silica gel TLC. As a result thereof, fractions developed on that chromatography indicated that free fatty acids, PC, LPC and MG were produced, and further indicated production of DG. Thus, it was confirmed that the culture supernatant, used as an enzyme solution, actually demonstrated phospholipase C activity, as a major enzymatic hydrolysis activity thereof.

Experiment 6: The Moritella sp. HFHI-0014 strain was inoculated in the K28 culture medium (containing 0.5 % of peptone, 0.1 % of yeast extract, and 50% of artificial seawater), and subjected to shaking cultivation for 72 hours at the temperature of 10 degrees Celsius. Centrifugation was carried out to the thus-cultivated medium for removal of the bacterial bodies therefrom, whereupon a culture supernatant without the bacterial bodies was obtained. As a substrate, 1.0 mg of 1-acyl lysophospholipid derived from soybean oil was provided. On the other hand, 0.5 ml of diethyl ether was added to 0.5 ml of the afore-said culture supernatant, thereby providing a total 1.0 ml of culture solution. Then, those substrate and culture solution were agitated together for 8 hours at the temperature of 10 degrees Celsius. A resultant solution obtained by such agitation was fractionated and analyzed by silica gel TLC. As a result thereof, fractions developed on that chromatography indicated that LPC was produced, and further indicated production of MG. Thus, it was confirmed that the culture supernatant, used as enzyme solution, actually demonstrated lysophospholipase C activity to act on the 1-acyl lysophospholipid.

Experiment 7: The Moritella sp. HFHI-0014 strain was inoculated in the K28 culture medium (containing 0.5 % of peptone, 0.1 % of yeast extract, and 50% of artificial seawater), and subjected to shaking cultivation for 72 hours at the temperature of 10 degrees Celsius. Centrifugation was carried out to the thus-cultivated medium for removal of the bacterial bodies therefrom, whereupon a culture supernatant without the bacterial bodies was obtained. As a substrate, 2.0 mg of sphingomyelin derived from egg yolk was provided. On the other hand, 0.5 ml of diethyl ether was added to 0.5 ml of the afore-said culture supernatant, thereby providing a total 1.0 ml of culture solution: Then, those substrate and culture solution were agitated together for 16 hours at the temperature of 10 degrees Celsius. A resultant solution obtained by such agitation was fractionated and analyzed by silica gel TLC. As a result thereof, fractions developed on that chromatography indicated that ceramide was produced. Thus, it was confirmed that the culture supernatant, used as an enzyme solution, actually demonstrated sphingomyelinase activity.

Accordingly, in accordance with the present invention, from all the results of Experiments 1 to 7 above, it is to be appreciated that the culture supernatant prepared from the Moritella sp. HFHI-0014 strain is provided with: phospholipase A₁ activity; phospholipase C activity; lysophospholipase C activity; and sphingomyelinase activity, while having neither of phospholipase A₂ activity and lipase activity, as shown in Table 6 below.

**Table 6**

| Enzymatic Activities of Moritella sp. HFHI-0014 Strain | | | | | | |
|---|---|---|---|---|---|---|
| Strain | PLA1 | PLA2 | PLC | LPLC | Lipase | SM Activity |
| HFHI-0014 | ○ | × | ○ | ○ | × | ○ |

| | | | | | | |
|---|---|---|---|---|---|---|
| * PLA1 ··· Phospholipase A₁ activity PLA2 ··· Phospholipase A₂ activity PLC ··· Phospholipase C activity LPLC ··· Lysophospholipase C activity SM Activity ··· Sphingomyelinase activity | | | | | | |

### Industrial Applicability of the Present Invention

The above-described culture solution in the present invention contains enzymes provided with phospholipase A₁ activity, phospholipase C activity, lysophospholipase C activity, and sphingomyelinase activity, and those enzymes may be isolated and refined into a state adaptable for industrial uses in emulsification of foods and cosmetics as well as for degumming processes.

## Claims

1. A novel microorganism belonging to Moritella species, which is **characterized by** being capable of producing enzymes provided with: phospholipase A₁ activity; phospholipase C activity; lysophospholipase C activity; and sphingomyelinase activity.

2. A novel microorganism belonging to Moritella species, which is **characterized by** being capable of producing enzymes provided with: phospholipase A₁ activity; phospholipase C activity; lysophospholipase C activity; and sphingomyelinase activity, and also **characterized by** having undermentioned bacterial properties as well as undermentioned physiological and biochemical properties:
A. Bacterial properties:
(1) Cell shape:
polymerismic rod bacteria of such dimensions that 1.0 to 1.2 µm x 2.0 to 3.0 µm
(2) Gram stainability: negative
(3) Presence of spores: negative
(4) Mobility: positive
(5) Colony state and shape (after 48 hours of culture time):
(a) Diameter: 1.0 to 2.0 mm
(b) Color tone: cream
(c) Shape: circular
(d) Topography: lens-shaped
(e) Fringe: whole border
(f) Surface shape: smooth
(g) Transparency: semi-transparent
(h) Viscosity: butter-like
(6) Growth temperature test (degree Celsius) :
(a) At 20 degrees Celsius: negative
(b) At 15 degrees Celsius: + w
(7) Catalase reaction: positive
(8) Oxidase reaction: positive
(9) Fermentation of glucose: positive
(10) O/F test (oxidation/fermentation): positive/positive
(11) Growth under aerobic conditions: positive
B. Physiological and biochemical properties:
(1) Nitrate reduction: positive
(2) Indole production: negative
(3) Glucose acidification: negative
(4) Arginine dihydrolase: negative
(5) Urease: negative
(6) Esculin hydrolysis: negative
(7) Gelatin hydrolysis: negative
(8) Beta galactosidase: negative
(9) Glucose: negative
(10) L-Arabinose: negative
(11) D-Mannose: positive
(12) Cellobiose: positive
(13) Starch hydrolysis: negative
(14) D-Mannitol: negative
(15) N-acetyl-D-glucosamine: negative
(16) Maltose: negative
(17) Potassium gluconate: negative
(18) n-Capric acid: negative
(19) Adipic acid: negative
(20) d1-Malic acid: negative
(21) Sodium citrate: negative
(22) Phenyl acetate: negative
(23) Cytochrome oxidase: positive
(24) Xylose: negative
(25) Glycerol: negative
(26) D-Galactose: negative

3. The novel microorganism as described in Claim 1 or 2, which is a microorganism belonging to Moritella species, said microorganism being assigned with a strain name of HFHI-0014 (FERM AP-20806).

4. A novel microorganism belonging to Moritella species, which is **characterized in that** a base sequence of 16Sr DNA gene of the novel microorganism is described in the sequence listing under sequence number 1.

5. A lipid-modifying agent **characterized by** containing effective dose of enzymes produced by said novel microorganism described in any one of Claims 1 to 4, wherein said enzymes are provided with one or at least two activities selected from the group consisting of: phospholipase A₁ activity; phospholipase C activity; lysophospholipase C activity; and sphingomyelinase activity.

6. The lipid-modifying agent as described in Claim 5, which is **characterized in that** a state of said enzymes for practical use is such that the enzymes are contained in bacterial bodies of said novel microorganism and/or in culture solution.

7. A method for producing 2-acyl lysophospholipid, which is **characterized by** hydrolyzing phospholipids by use of an enzyme provided with phospholipase A₁ activity, to thereby obtain the 2-acyl lysophospholipid, wherein said enzyme is produced by said novel microorganism described in any one of Claims 1 to 4.

8. The method for producing 2-acyl lysophospholipid as described in Claim 7, which is **characterized in that** a state of said enzyme for practical use is such that the enzyme is contained in bacterial bodies of said novel microorganism and/or in culture solution.

9. A method for producing diacylglycerol, which is **characterized by** hydrolyzing phospholipids by use of enzymes produced by the novel microorganism described in any one of Claims 1 to 4, to thereby obtain the diacylglycerol.

10. The method for producing diacylglycerol as described in Claim 9, which is **characterized in that** a state of said enzymes for practical use is such that the enzymes are contained in bacterial bodies of said novel microorganism and/or in culture solution.

11. A method for producing ceramide, which is **characterized by** hydrolyzing phospholipids by use of enzymes produced by said novel microorganism described in any one of Claims 1 to 4, to thereby otabin the ceramide.

12. The method for producing ceramide as described in Claim 11, which is **characterized in that** a state of said enzymes for practical use is such that the enzymes are contained in bacterial bodies of the novel microorganism and/or in culture solution.

13. A method for degumming fat and oil, which is **characterized by** hydrolyzing phospholipids present in the fat and oil, by use of enzymes produced by said novel microorganism described in any one of Claims 1 to 4, to thereby degum the fat and oil.

14. The method for degumming fat and oil as described in Claim 13, which is **characterized in that** a state of said enzymes for practical use is such that the enzymes are contained in bacterial bodies of the novel microorganism and/or in culture solution.
